# EUROPEAN PATENT APPLICATION

(11) **EP 3 943 112 A2**
(43) Date of publication of application: **26.01.2022**
(21) Application number: 20772922.9
(22) Date of filing: 09.03.2020
(51) Int. Cl.: A61K 47/54, A61K 31/496, A61P 35/00, C07F 9/6561

(54) **REPURPOSED ANTIBIOTICS FOR NON-NUCLEAR GENOTOXIC CHEMOTHERAPY AND PHARMACEUTICAL COMPOSITION FOR ANTI-CANCER CONTAINING THE SAME**

(30) Priority: 18.03.2019 KR 20190030384
(71) Applicant: Korea University Research and Business Foundation, Seoul 02841 (KR)
(72) Inventor: KIM, Jong Seung, Seoul 02841 (KR); SUNWOO, Kyoung, Seoul 02841 (KR); VERWILST, Peter, Seoul 02841 (KR); WON, Miae, Seoul 02841 (KR)
(74) Representative: Hoefer & Partner Patentanwälte mbB
(86) International application number: PCT/KR2020/003248
(87) International publication number: WO 2020/189937

(57) **Abstract**

The present invention relates to a repurposed antibiotic compound for the treatment of cancer with minimal nuclear gene damage and an anticancer pharmaceutical composition comprising same. Since the repurposed antibiotic compound has a therapeutic effect in a manner that targets only the mitochondria of cancer cells, the modified antibiotic compound does not cause gene degeneration unlike conventional chemotherapy which damages nuclear DNAs to kill cancer cells, , thereby preventing the recurrence of cancer. In addition, a mitochondria targeted therapy using the compound according to the present invention can effectively treat malignant tumors that are difficult to treat due to acquiring drug resistance by general anticancer treatment.

## Description

### Technical Field

The present invention relates to a modified antibiotic compound for cancer therapy with minimal damage to nuclear genes and an anticancer pharmaceutical composition including the same.

### Background Art

Numerous research groups around the world have made efforts to develop various drug delivery systems for improving the therapeutic effects of existing anticancer drugs with reduced side effects, and as a result, succeeded in developing drug delivery systems that effectively deliver existing highly toxic anticancer drugs to cancer tissues and allow the anticancer drugs to be activated only in the cancer microenvironment, achieving significantly improved safety in cancer treatment.

Despite these technological advances, however, the treatment and suppression of recurrent and metastatic cancers with acquired drug resistance remain challenges for conventional anticancer therapies. 90% of cancer deaths are caused by recurrent cancers, not primary cancers. People who have had cancer have a very high probability of cancer recurrence throughout their lives even after 5 years of survival. Especially, pediatric cancer patients have a more increased risk of cancer recurrence. It has recently been reported that cancer recurrence is paradoxically due to the use of anticancer drugs.

General chemotherapy inhibits the replication of nuclear DNA in cancer cells and causes nuclear DNA damage to kill cancer cells. In this course, mutations occur and will cause cancer recurrence. It was found that the administration of temozolomide to stage 2 glioma patients for anticancer therapy led to more malignant (stage 4) glioma as a recurrent cancer and DNA mutations induced by the anticancer drug temozolomide were detected in the recurrent cancer.

Mitochondria are intracellular organelles that have their own circular DNA different from the nucleus in animals and divide and fuse in the same way as prokaryotic cells. For these reasons, the origin of mitochondria is explained by the endosymbiotic theory in which bacteria invaded host cells during evolution and became mitochondria in animal cells. The evidence is that mitochondria have their own circular DNA, like bacteria, and are structurally and genetically similar to bacteria. Much research revealed that since mitochondria use enzymes for biosynthesis, like bacteria, some antibiotics primarily targeting bacteria inhibit mitochondrial function to cause mitochondrial toxicity, which is responsible for their side effects. Attempts have been made to kill cancer cells by reversely using the toxicity of the antibiotics. However, the antibiotics do not exhibit significant toxicity to human cells at low concentrations and inhibit the division of both cancer cells and normal cells (but even they are more selectively toxic to normal cells) at high concentrations, making their use as anticancer drugs substantially impossible. That is, the research results are simply limited to the toxicity of the antibiotics to animal cells. Some studies have also revealed that treatment of cancer stem cells with the antibiotics at high concentrations inhibits cancer growth. However, the antibiotics have no effect on bulk cancer cells, which account for 99% of the total cancer volume, and inhibit the division and growth of cancer stem cells rather than kill cancer stem cells. That is, these studies simply suggest possible therapeutic effects of the antibiotics.

Cancer stem cells with high mitochondrial mass and high mitochondrial membrane potential can be most effectively eliminated by drug delivery systems that use the mitochondrial membrane potential to deliver and accumulate drugs in mitochondria. Particularly, selective targeting of mitochondria in cancer cells for effective cancer treatment would prevent cancer recurrence without causing genetic modification, unlike conventional chemotherapy for killing cancer cells by nuclear DNA damage.

### Detailed Description of the Invention

### Problems to be Solved by the Invention

The present invention intends to provide a modified antibiotic anticancer drug that selectively kills cancer cells and cancer stem cells without damage to normal cells and normal stem cells, and after the lapse of a predetermined time, loses its targeting group to become a simple antibiotic drug that can be safely released from the body without secondary toxicity, thus being useful for a novel cancer therapeutic strategy to inhibit cancer recurrence and metastasis and minimize the possibility of possible side effects.

### Means for Solving the Problems

One aspect of the present invention provides a modified antibiotic compound for cancer therapy with minimal damage to nuclear genes, represented by Formula 1:

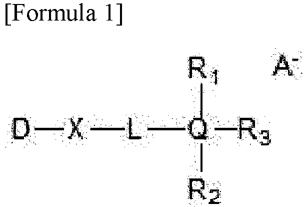

The structure and substituents of the antibiotic represented by Formula 1 will be described below.

The modified antibiotic anticancer compound of the present invention minimizes damage to nuclear genes in cancer cells and selectively targets mitochondria in cancer cells and thus it inhibits the mitochondrial electron transport chain (ETC) and mitochondrial DNA synthesis in cancer cells.

A further aspect of the present invention provides a pharmaceutical composition for preventing and treating cancer diseases with minimal damage to nuclear genes, including a modified antibiotic compound represented by Formula 1 or a salt thereof as an active ingredient.

### Effects of the Invention

The modified antibiotic anticancer drug of the present invention selectively targets mitochondria in cancer cells to exhibit its therapeutic effect. Thus, the modified antibiotic anticancer drug of the present invention can prevent cancer recurrence without causing genetic modification, unlike conventional chemotherapy for killing cancer cells by nuclear DNA damage. In addition, the compound of the present inv ion can be used to provide a mitochondria-targeted therapy for effective treatment of malignant tumors that are difficult to treat by general anticancer therapies due to their acquired drug resistance.

### Brief Description of the Drawings

Fig. 1 shows the viabilities of metastatic breast cancer cells (MDA-MB-231) after treatment with a compound (Ester Mt-CFX) of the present invention and a commercial antibiotic (ciprofloxacin).
Fig. 2 shows the viabilities of metastatic breast cancer cells (MDA-MB-231) after treatment with Ester Mt-CFX and Amide Mt-CFX, which was obtained by replacing the binding site of Ester Mt-CFX with an amide group for better *in vivo* compatibility.
Fig. 3 shows the viabilities of various cancer cell types, including A549 (lung cancer cells), SW620 (colon cancer cells), DU145 (prostate cancer cells) and PC3 (prostate cancer cells), after treatment with a compound (Ester Mt-CFX) of the present invention.
Fig. 4 shows the production of reactive oxidative species (ROS) in cells by a compound (Ester Mt-CFX) of the present invention, which was labeled with fluorescent markers (Amplex-red and CM-H2DCFDA).
Fig. 5 shows the inhibition of mitochondrial DNA synthesis and the reduction of membrane potential after treatment with a compound (Ester Mt-CFX) of the present invention.
Fig. 6 shows the degrees of damage to DNA, protein, and lipid by reactive oxygen species generated after treatment of a compound (Ester Mt-CFX) of the present invention.
Fig. 7 shows the reduction of mitochondrial membrane potential differences (JC-1 assay) and the occurrence of apoptosis (Annexin V assay) overtime after treatment with a compound (Ester Mt-CFX) of the present invention.
Fig. 8 compares the degrees of damage to nuclear DNA and mitochondrial DNA by an anticancer compound (Ester Mt-CFX) of the present invention and a commercial anticancer drug (doxorubicin (DOXO)).
Fig. 9 shows the expression levels of DNA repair proteins after treatment with a compound (Ester Mt-CFX) of the present invention and a commercial anticancer drug (doxorubicin (DOXO)) (where POLy is a protein involved in mitochondrial gene repair and ERCC1 and DDB2 are proteins involved in nuclear gene repair).
Fig. 10 compares the degrees of damage to mitochondrial genomic DNA by Ester Mt-CFX and conventional anticancer drugs causing damage to nuclear DNA, which were determined by Taq1 assay.
Fig. 11 shows the results of real-time PCR for nuclear and mitochondrial DNA damage lesions after treatment with a compound (Ester Mt-CFX) of the present invention and conventional commercial anticancer drugs (doxorubicin, emozolomide, camptothecin, cisplatin, chlorambucil, and 5-FU).
Fig. 12 shows the inhibitory effect of a compound (Ester Mt-CFX) on tumor cells using breast cancer transplantation models by determining tumor-targeting effects over time (*in vivo* imaging) and measuring time-dependent changes in tumor volume and weight after injection of the compound.

### Best Mode for Carrying out the Invention

The present invention will now be described in more detail.

The present invention is directed to a modified antibiotic drug for a new concept of anticancer therapy that can inhibit recurrence and metastasis contributed from strong genotoxicity which is a major problem of current anticancer therapies.

The modified antibiotic anticancer drug compound of the present invention is represented by Formula 1: 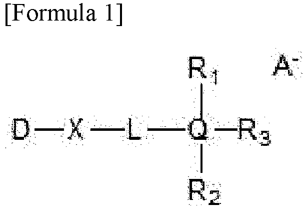 wherein D is the backbone of a highly stable fluoroquinolone antibiotic selected from flumequine, oxolinic acid, rosoxacin, cinoxacin, nalidixic acid, piromidic acid, pipemidic acid, ciprofloxacin, fleroxacin, lomefloxacin, nadifloxacin, norfloxacin, ofloxacin, pefloxacin, rufloxacin, enoxacin, balofloxacin, grepafloxacin, levofloxacin, pazufloxacin sparfloxacin, temafloxacin, tosufloxacin, clinafloxacin, gatifloxacin, moxifloxacin, sitafloxacin, prulifloxacin, besifloxacin, gemifloxacin, trovafloxacin, delafloxacin, danofloxacin, difloxacin, enrofloxacin, ibafloxacin, marbofloxacin, orbifloxacin, and sarafloxacin; X is connected to the terminal hydroxyl group of the backbone of the fluoroquinolone antibiotic and is selected from O, S and NR (wherein R is selected from hydrogen, C₁-C₃₀ alkyl groups, C₆-C₃₀ aryl groups, and C₂-C₃₀ heteroaryl groups), preferably O or NR; L is a linking group with the targeting group and is selected from C₁-C₃₀ alkyl groups, C₂-C₃₀ alkenyl groups, and polyalkylene glycol groups; Q is a Group 15 element selected from N, P, As and Sb; R₁ to R₃ are the same as or different from each other and are each independently selected from C₁-C₃₀ alkyl groups, C₂-C₃₀ alkenyl groups, C₆-C₃₀ aryl groups, and C₂-C₃₀ heteroaryl groups; and A⁻ is an anion selected from halogen, hydroxyl, carboxylate, sulfate, sulfamate, sulfonate, phosphate, phosphonate, boronate, and (poly)ethyleneoxy anions.

The modified antibiotic anticancer drug compound of the present invention uses the backbone of a highly stable antibiotic as a basic structure and is designed to selectively target mitochondria, achieving enhanced anticancer therapeutic efficacy. In addition, the modified antibiotic anticancer drug compound of the present invention becomes a safe antibiotic drug after the mitochondria-targeting group is hydrolyzed *in vivo,* causing minimal side effects. When X is an amide group (NR), better *in vivo* stability of the compound represented by Formula 1 is ensured.

According to one exemplary embodiment of the present invention, the compound of Formula 1 is selected from, but not limited to:

[Ester Mt-CFX] 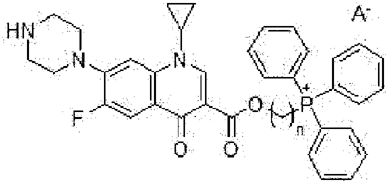 wherein n is an integer from 1 to 30 and A⁻ is as defined in Formula 1; and

[Amide Mt-CFX] 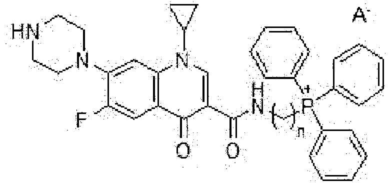 wherein n and A⁻ are as defined above.

The compound of Formula 1 has the backbone of ciprofloxacin as a fluoroquinolone antibiotic.

In the Examples section that follows, n is 6 and A⁻ is a chloride anion. Amid Mt-CFX is a compound obtained by replacing the binding site of Ester Mt-CFX with an amide group for better *in vivo* stability.

The present invention is also directed to a pharmaceutical composition for preventing and treating cancer diseases, including the modified antibiotic anticancer compound represented by Formula 1 or a salt thereof as an active ingredient.

The pharmaceutical composition of the present invention can be used to prevent and treat a wide range of cancer diseases such as primary cancers and their metastatic cancers. Examples of the cancer diseases include, but are not limited to, breast cancer, lung cancer, colon cancer, prostate cancer, and metastatic cancers thereof.

The pharmaceutical composition of the present invention may be complexed with other known drugs for the prevention or treatment of cancer diseases before administration or may further include one or more other additives selected from carriers, diluents, adjuvants and stabilizers.

The dosage form of the composition according to the present invention may vary depending on the mode of administration desired. Examples of such dosage forms include, but not limited to, solid, semi-solid, and liquid formulations such as tablets, pills, powders, capsules, gels, ointments, emulsions, and suspensions. The composition of the present invention may be administered in unit dosage forms suitable for single administration of precise dosages. The composition of the present invention may be administered orally or parenterally. For parenteral administration, the composition of the present invention may be administered intravenously, subcutaneously or intramuscularly.

Depending on the formulation desired, the composition may further include one or more pharmaceutically acceptable carriers, diluents, adjuvants, and stabilizers, which are defined as aqueous-based vehicles commonly used to formulate pharmaceutical compositions for human administration.

The rm "carrier" means a substance that facilitates the incorporation of a compound into cells or tissues. Examples of suitable carriers include, but not limited to, carbohydrate-based compounds, such as lactose, amylose, dextrose, sucrose, sorbitol, mannitol, starch, and cellulose, gum acacia, calcium phosphate, alginate, gelatin, calcium silicate, microcrystalline cellulose, polyvinylpyrrolidone, cellulose, water, syrups, salt solutions, alcohols, gum Arabic, vegetable oils, such as corn oil, cotton seed oil, soybean oil, olive oil, and coconut oil, polyethylene glycol, methyl cellulose, methyl hydroxybenzoate, propyl hydroxybenzoate, talc, magnesium stearate, and mineral oils, which are commonly used to formulate pharmaceutical compositions. The rm "diluent" is defined as a substance diluted in water that can dissolve the compound of i erest as well as stabilize the biologically active form of the compound. Examples of suitable diluents include distilled water, physiological saline, Ringer's solution, dextrose solution, and Hank's solution. The stabilizers can be selected from the group consisting of proteins, carbohydrates, buffers, and mixtures thereof. The composition of the present invention may optionally further include one or more additives. Examples of such optional additives include, but not limited to, lubricating agents, wetting agents, sweetening agents, flavoring agents, emulsifying agents, suspending agents, and preservatives.

Such additional addi ives as carriers, diluents, adjuvants, and stabilizers may be used in amounts effective to acquire pharmaceutically acceptable formulations in view of the solubility, biological activity, and other characteristics of the active ingredient.

### Mode for Carrying out the Invention

The present invention will be more specifically explained with reference to the following examples. It will be appreciated by those skilled in the art that these examples are merely illustrative and the scope of the present invention is not limited thereto.

### Synthesis Example 1: Synthesis of Ester Mt-CFX

Ester Mt-CFX was synthesized according to Scheme 1:

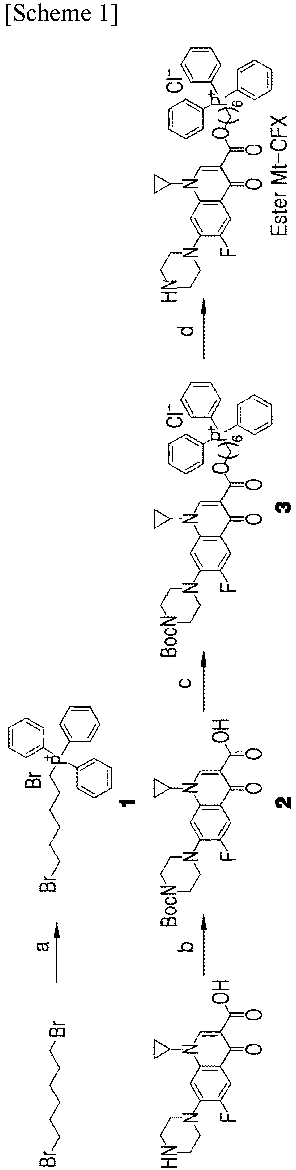

Compounds 1 and 2 shown in Scheme 1 were synthesized according to methods known in the art.

### (1) Synthesis of Compound 3

Compound **1** (1 g, 1.975 mmol) and Compound **2** (852 mg, 1.975 mmol) were dissolved in dimethylformamide as a solvent, and then potassium carbonate (819 mg, 5.925 mmol) was slowly added thereto. The mixture was stirred at 50 °C for 12 h. The reaction mixture was evaporated under reduced pressure to remove the solvent. The residue was purified by column chromatography and dissolved in 50 mL of a mixed solution of methanol and distilled water (1/9). NaBF₄ was added to the solution, followed by stirring for 1 h. The reaction mixture was extracted with dichloromethane and distilled water and dissolved in methanol. After addition of Dowex^{®} 1×8 chloride, he resulting mixture was stirred for 6 h. The reaction mixture was filtered and evaporated to remove the solvent.

### (2) Synthesis of Ester Mt-CFX

Compound **3** was dissolved in 18 mL of a mixed solution of 1,4-dioxane and dichloromethane (4/1), and then 6 mL of 4 N HCl in dioxane was added dropwise thereto at 0 °C. The mixture was sti ed at room temperature for 12 h. The solvents were removed by evaporation under reduced pressure, affording Ester Mt-CFX.

¹H NMR (CDCl₃, 500 MHz): δ 8.50 (s, 1H), 7.87-7.70 (m, 15H), 7.66 (d, = 13.1 Hz, 1H), 7.36 (d, *J* = 7.1 Hz, 1H), 4.29-4.23 (m, 2H), 3.69-3.60 (m, 2H), 3.59-3.53 (m, 1H), 3.50-3.42 (m, 4H), 3.30-3.20 (m, 4H), 1.78-1.65 (m, 6H), 1.61-1.53 (m, 2H), 1.39 (d, *J* = 5.9 Hz, 2H), 1.14-1.07 (m, 2H) ppm.

### Synthesis Example 2: Synthesis of Amide Mt-CFX

Amide Mt-CFX was synthesized according to Scheme 2:

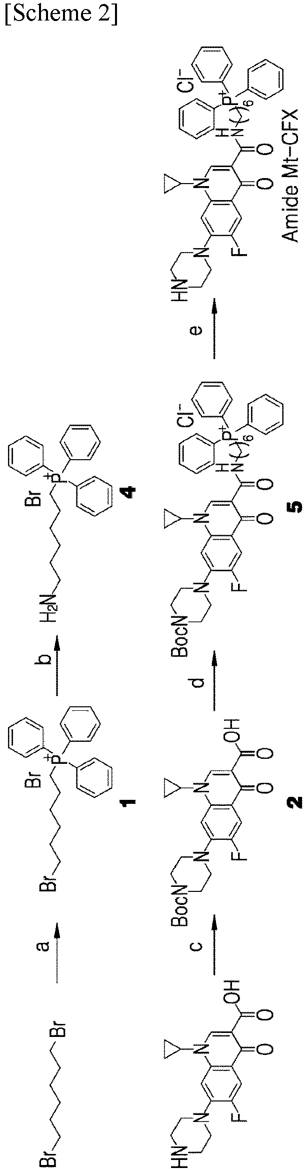

Compounds **1** and **2** shown in Scheme 2 were synthesized according to methods known in the art.

### (1) Synthesis of Compound 4

Compound **1** (1 g, 1.975 mmol) was dissolved in 35 mL of a solution of 7 N NH₃ in methanol. The solution was stirred at room temperature for 3 days. Thereafter, the reaction solution was evaporated under reduced pressure to remove the solvent. The residue was purified by column chromatography.

### (2) Synthesis of Compound 5

Compound **2** (1 g, 2.318 mmol), EDC hydrochloride (667 mg, 3.477 mmol), and 1-hydroxybenzotriazole hydrate (470 mg, 3.477 mmol) were dissolved in dimethylformamide as a solvent. The solution was stirred at room temperature for 30 min. To the reaction solution were added DMAP (425 mg, 3.477 mmol) and Compound **4** (1.025 g, 2.318 mmol). The resulting mixture was stirred for 12 h. The reaction mixture was evaporated under reduced pressure to remove the solvent. The residue was purified by column chromatography and dissolved in 50 mL of a mixed solution of methanol and distilled water (1/9). NaBF₄ was added to the solution, followed by stirring for 1 h. The reaction mixture was extracted with dichloromethane and distilled water and dissolved in methanol. After addition of Dowex^{®} 1×8 chloride, the resulting mixture was stirred for 6 h. The reaction mixture was filtered and evaporated to remove the solvent.

### (3) Synthesis of Amide Mt-CFX

Compound **5** was dissolved in 18 mL of a mixed solution of 1,4-dioxane and dichloromethane (4/1), and then 6 mL of 4 N HCl in dioxane was added dropwise thereto at 0 °C. The mixture was stirred at room temperature for 12 h. The solvents were removed by evaporation under reduced pressure, affording Amide Mt-CFX.

¹H NMR (MeOD, 500 MHz): δ 8.88 (s, 1H), 7.95-7.74 (m, 17H), 7.69 (d, *J* = 7.2 Hz, 1H) 3.85-3.79 (m, 1H), 3.69-3.64 (m, 4H), 3.54-3.49 (m, 4H), 3.49-3.41 (m, 4H), 1.77-1.70 (m, 2H), 1.70-1.58 (m, 4H), 1.53-1.42 (m, 4H) ppm.

### Experimental Example

### 1-1. Animal cell culture

Human metastatic breast cancer cell line MDA-MB-231 (human breast cancer cells), lung cancer cell line A549 (human lung carcinoma cells), human colon cancer cell line SW620 (human colon carcinoma cells), human prostate cancer cell lines DU145 and PC3 (human prostate cancer cells) were cultured in RPMI1640 media and modified Eagle's media (MEM). All media were supplemented with 10% inactivated fetal bovine serum (FBS) and 1% penicillin-streptomycin. All cell lines were cultured at 37 °C and 5% carbon dioxide.

### 1-2. Cell viability analysis

MDA-MB-231, A549, SW620, DU145, and PC3 cells were seeded into 96-well plates and cultured overnight for stabilization. After treatment of the stabilized cells with Ester MT-CFX, Amide MT-CFX, and ciprofloxacin at various concentrations from 0 to 100 µM for 48 h, the amounts of LDH in living cells were measured to determine cell viabilities. Data are shown as the mean ± SE of three independent experiments.

### 1-3. Reactive oxidative species (ROS) measurement and analysis

The activity of hydrogen peroxide (H₂O₂) produced in cells was evaluated by detection with Amplex^{®} Red reagent (10-acetyl-3,7-dihydroxyphenoxazine; Thermo Fisher scientific). Here, Amplex^{®} Red reagent reacts with H₂O₂ in a stoichiometric ratio of 1:1 in the presence of peroxidase in cells to produce resorufin as a red fluorescent product. The fluorescence of resorufin can be analyzed at excitation and emission wavelengths of 571 nm and 581 nm, respectively. The MDA-MB-231 cell line was seeded into a 96-well plate and treated with 10 µM Ester MT-CFX for 48 h. Thereafter, the amount of hydrogen peroxide was measured. A reaction mixture of 50 µM Amplex^{®} Red reagent and 0.1 U/mL HRP was prepared in Krebs-Ringer phosphate buffer (145 mM NaCl, 5.7 mM sodium phosphate, 4.86 mM KCl, 0.54 mM CaCl₂, 1.22 mM MgSO₄, 5.5 mM glucose, pH 7.35). 100 µL of the reaction mixture was added to each plate well, followed by incubation at 37 °C for 10 min. The brightness of fluorescence was measured at 530-560 nm excitation and 590 nm emission wavelengths using a microplate reader. The MDA-MB-231 cell line was seeded into a confocal dish. Cells were treated with 10 µM Ester MT-CFX for 48 h, followed by incubation with 10 µM CM-H₂DCFDA dye for 30 min. CM-H₂DCFDA dye is a known marker for reactive oxygen species in cells. The fluorescence of reactive oxygen species produced in cells was imaged by confocal laser scanning microscopy at 492-495 nm excitation and 517-527 nm emission wavelengths.

### 1-4. DNA oxidation measurement

Intracellular DNA oxidation by oxidative stress was analyzed by enzyme-linked immunoassay using a commercially available 8-OHdG ELISA kit. After treatment of the MDA-MB-231 cells with 10 µM Ester MT-CFX and 10 µM CFX for 48 h, the samples were incubated with primary antibodies at 37 °C and secondary antibodies at 37 °C for 1 h. The incubated samples were allowed to stand at room temperature for 15 min for color development. The absorbance values of the resulting solutions were measured at 450 nm using a multiplate reader.

### 1-5. Protein carbonylation measurement

Protein carbonyl groups are known to be important physiological markers of oxidative stress and reactive oxygen species (ROS) are liable to damage intracellular proteins. Protein carbonylation was determined by ELISA. After treatment of MDA-MB-231 cells with 10 µM Ester MT-CFX and 10 µM CFX for 48 h, 10 µL of 1 µg/µL of protein lysate from each sample was denatured with 10 µL of 10% (w/v) SDS and derivatized with 20 µL of 20 mM 2,4-dinitrophenylhydrazine (DNPH) solution prepared in TPA. After incubation at room temperature for 10 min with vortexing every 2 min, the reaction product was neutralized with 20 µL of 2 M Tris-Cl. A 3 µL aliquot of DNP-derivatized sample was diluted with 0.25 mL of adsorption buffer (20 mM NaHCO₃, 150 mM NaCl, 0.25% SDS, pH 8.5), and 100 µL of diluted sample was loaded on to a 96-well plate. The plate was covered with aluminum foil and incubated overnight at 4 °C. After incubation, the sample wells were rinsed 5 times with PBST (0.5% Tween 20) and incubated with 200 µL of blocking buffer (1% BSA in adsorption buffer) at 37 °C for 1 h. The sample wells were then incubated with 100 µL of blocking buffer containing goat anti-DNP antibody at room temperature for 1 h. Following incubation, the sample wells were rinsed 5 times with PBST, and incubated with HRP-conjugated rabbit anti-goat IgG antibody at room temperatur for 1 h. After washing 5 times with PBST, standard wells were incubated with 100 µL of TMB substrate at room temperature for 2-3 min for color development. The reaction was stopped with 100 µL of 0.5 M H₂SO₄ and the absorbance was measured at 450 nm and 690 nm. After subtraction of background absorbance at 690 nm, the carbonyl content in each sample was determined using a standard curve of oxidized BSA standard.

### 1-6. Lipid peroxidation measurement

Lipid oxidation is one of the markers for the mechanism of cell damage and can be confirmed by measuring malondialdehyde (MDA), a product of lipid peroxidation. After treatment of the MDA-MB-231 cells with 10 µM Ester MT-CFX and 10 µM CFX for 48 h, 1 mg/mL of each sample was placed in phosphate buffer and incubated in a thermostatic bath at 37 °C for 6 h. 0.5 mL of 0.75% thiobarbituric acid was added to 10 µL of a mixed solution of butylated hydroxytoluene (BHT) quenched with 10% TCA. The mixture was heated at 95 °C for 20 min, cooled and centrifuged at 780×g for 10 min. The absorbance of the resulting solution was measured at 532 nm using a multiplate reader.

### 1-7. FACS measurement

Annexin-V positive apoptotic cells were analyzed by flow cytometry. To this end, the MDA-MB-231 cells were treated with 10 µM Ester MT-CFX for different periods of time (0, 6, 12, and 24 h) and cultured for indicated periods of time. Cells were harvested, washed twice with ice-cold PBS, placed in BD FACS tubes (10⁶/mL) and cultured with 10 µg/mL annexin V in PBS containing 10% FBS at room temperature for 30 min. Subsequently, cells were washed with ice-cold PBS and annexin V fluorescence was measured in the FL-1 channel using a FACSCalibur flow cytometer (BD, USA).

### 1-8. Mitochondrial membrane potential measurement

Mitochondrial membrane potential differences were measured. To this end, the

MDA-MB-231 cells were treated with 10 µM Ester MT-CFX for different periods of time (0, 6, 12, and 24 h) and cultured for indicated periods of time. Cells were harvested, washed once with ice-cold PBS, and cultured with 2 M 5,5',6,6'-tetrachloro-1,1',3,3'-traethyl-benzimidazolyl carbocyanine chloride (JC-1) at 37 °C for 20 min. The JC-1 stained cells were centrifuged at 1,300 g for 3 min and washed twice with ice-cold PBS. Mitochondrial membrane potentials of JC-1 monomer (green channel) and aggregate (red channel) were measured in different wavelength bands.

### 1-9. Real-time PCR analysis

RNA was extracted from each sample using TRIzol solution. cDNA synthesized from the isolated RNA or intracellular genomic DNA extracted using a genomic DNA isolation kit was used for real-time polymerase chain reaction (PCR). The real-time PCR data were normalized by the expression of glyceraldehyde-3-phosphate dehydrogenase (GAPDH). Relative expression levels were expressed as fold changes over the control. The results (mean ± SE) were obtained in triplicate and ap value < 0.05 was taken to be statistically significant.

### 1-10. Animal reparation

Five-week-old immunodeficient male BALB/c-nu-nu mice (Orient bio, Korea) weighing between 18-20 g were used. The animals were bred and housed in a facility maintaining a humidity of 30-40% and a temperature of 22 ± 1 °C on a 12-h light/dark cycle. All animal experiments were approved by the Ethics Committee for Animal Studies at Korea University. Animals were treated in accordance with the protocol approved by the Ethics Committee.

### 1-11. In vivo mouse xenograft models

MDA-MB-231 cells (5×10⁶ cells) were injected subcutaneously into 6-week-old immunodeficient BALB/c-nu-nu mice weighing between 18-20 g to establish tumor-bearing mouse models. Ciprofloxacin and Ester MT-CFX were injected intravenously into the established tumor-bearing mouse models three times a week. The lengths of the long and short ax s of tumors were measured weekly. 12 weeks after the first injection of each compound, the mice were sacrificed and tumor samples were collected for further analysis. The results are shown as the mean ± SE of the tumors of 4 mice in each group. Each asterisk (*) indicates a significant difference to the corresponding control.

### 1-12. In vivo fluorescence imaging

The tumor-targeting ability of Ester MT-CFX and the distribution of Ester MT-CFX in each organ were evaluated. Using an IVIS Lumina Series III Preclinical imaging system (PerkinElmer CO., USA), *in vivo* spectral fluorescence images were obtained from 1 h to 48 h after injection of 2 µmοl/kg of Ester MT-CFX into tumor-bearing mice via the tail vein. Filt s used to obtain the *in vivo* images were measured at an excitation wavelength of 560 nm. The fluorescence images were deconvoluted using the multi-excitation spectral analysis function.

### 1-13. Statistical analysis

The mean and standard error (SE) of each group were calculated from three independent experiments done in triplicate. Statistically significant differences between groups were evaluated by one-way analysis of variance (ANOVA) using SAS software (version 9.0, Cary). When the ANOVA showed a significant difference, comparisons of group means were performed using Student's t-tests. A value < 0.05 was taken to be statistically significant.

### Experimental Results

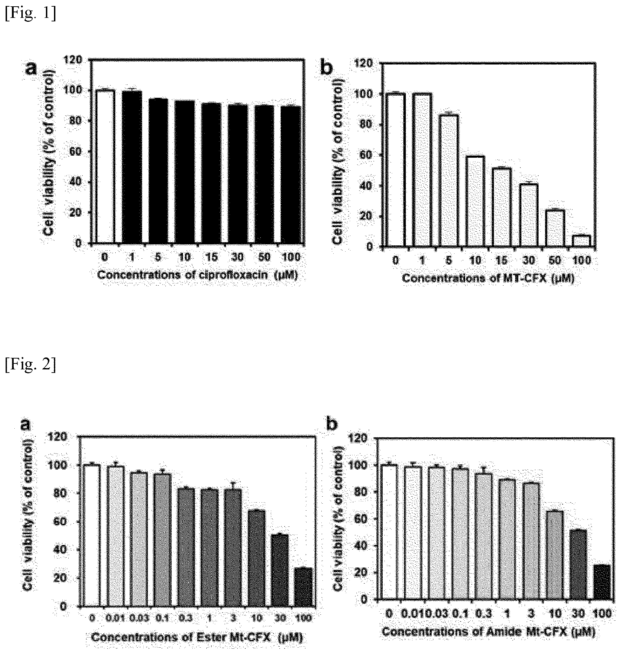

(1) The commercially available antibiotic ciprofloxacin has little or no apoptotic effect on MDA-MB-231 metastatic breast cancer cells. In contrast, the inventive compound Ester Mt-CFX having a targeting group showed a strong apoptotic effect on MDA-MB-231 cells with an IC₅₀ of ~15 µM (Fig. 1).

Fig. 1 shows the viabili ies of MDA-MB-231 tumor cell line (Korean Cell Line Bank). MDA-MB-231 is a triple-negative breast cancer cell line. Currently, there is no anticancer drug that can effectively treat triple-negative breast cancer. Cells were cultured in DMEM supplemented with 10% FBS at 37 °C and 5% CO₂. Cytotoxicity was determined by LDH assay. Cells were cultured in a dish for -24 h such that they remained stably adherent to the bottom of the dish, followed by treatment with the antibiotic ciprofloxacin and the inventive Ester Mt-CFX for 48 h. As a result of repeated experiments, the IC₅₀ of ciprofloxacin was estimated to be 1.65 M and that of Ester Mt-CFX was 20-30 µM, demonstrating that the apoptotic activity of Ester Mt-CFX against the cancer cells was ~55,000-fold higher than that of ciprofloxacin. In conclusion, the inventive anticancer drug has an excellent anticancer effect on triple-negative breast cancer.

(2) For better *in vivo* stability, Amide Mt-CFX was synthesized by converting the binding site (ester group) of Ester Mt-CFX to an amide group. The apoptotic effect of Amide Mt-CFX on cancer cells was verified to be similar to that of Ester Mt-CFX (Fig. 2).

The same experimental procedure as described for Ester Mt-CFX (Fig. 1) was repeated for the inventive Amide Mt-CFX. The results are shown in Fig. 2. The inventive Amide Mt-CFX also showed an enhanced apoptotic effect on cancer cells.

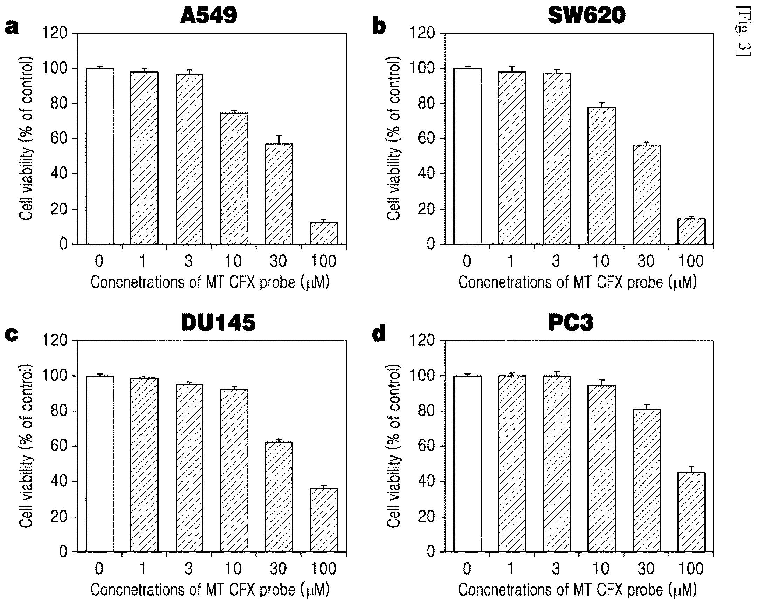

(3) The inventive compounds Ester Mt-CFX and Amide Mt-CFX showed apoptotic effects on various cancer cell types, including A549 (lung cancer cells), SW620 (colon cancer cells), DU145 (prostate cancer cells), and PC3 (prostate cancer cells) as well as MDA-MB-231 metastatic breast cancer cells, demonstrating their wide applicability (Fig. 3).

To demonstrate the applicability of the inventive anticancer drugs to various cancer cell types, the same experimental procedure as described for MDA-MB-231 metastatic breast cancer cells (Fig. 1) was repeated for A549, DU145, SW620, and PC3 tumor cell lines (Korean Cell Line Bank). The results are shown in Fig. 3. The inventive anticancer drugs were found to be effective against various cancer cell types. Particularly, the inventive anticancer drugs had IC₅₀ values of 23.77 µM and 27.05 µM against A549 and SW620, respectively.

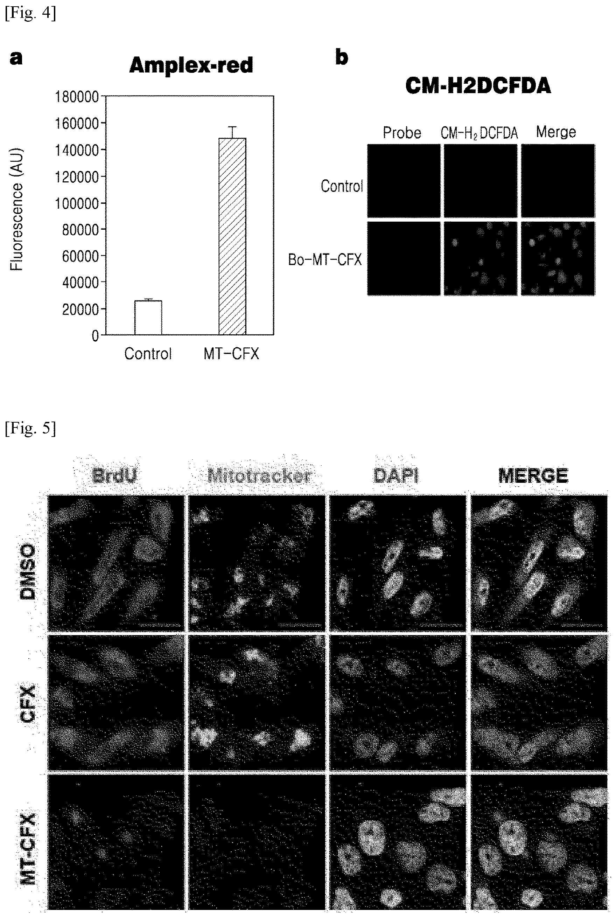

(4) The anticancer effect of the inventive modified antibiotic Mt-CFX can be explained by two mechanisms: (i) the accumulation of the compound Mt-CFX in mitochondria to inhibit the electron transport chain (ETC), as a result of which reactive oxygen species are produced (Fig. 4), and (ii) the inhibition of mitochondrial DNA synthesis (Fig. 5).

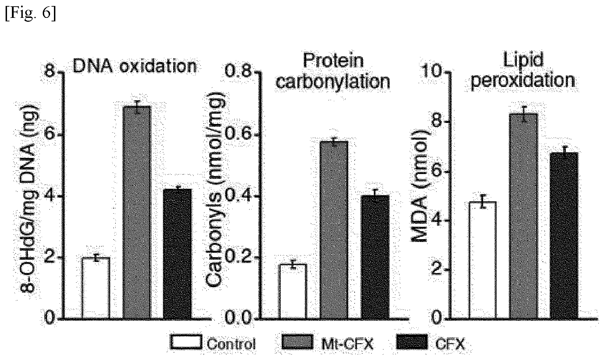

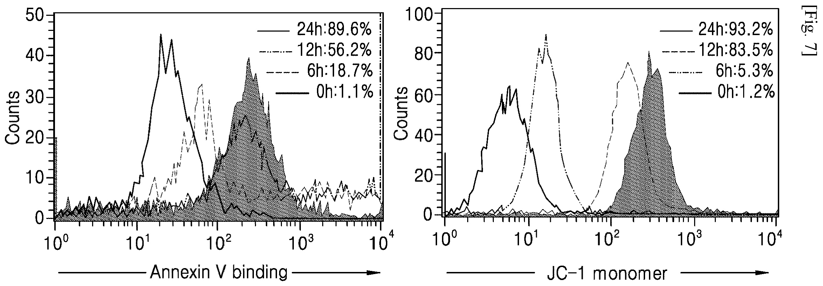

Fig. 6 shows the degrees of damage to DNA, protein, and lipid by reactive oxygen species generated after treatment of the inventive compound Ester Mt-CFX. Fig. 7 shows the reduction of mitochondrial membrane potential differences (JC-1 assay) and the occurrence of apoptosis over time after treatment with the inventive compound Ester Mt-CFX.

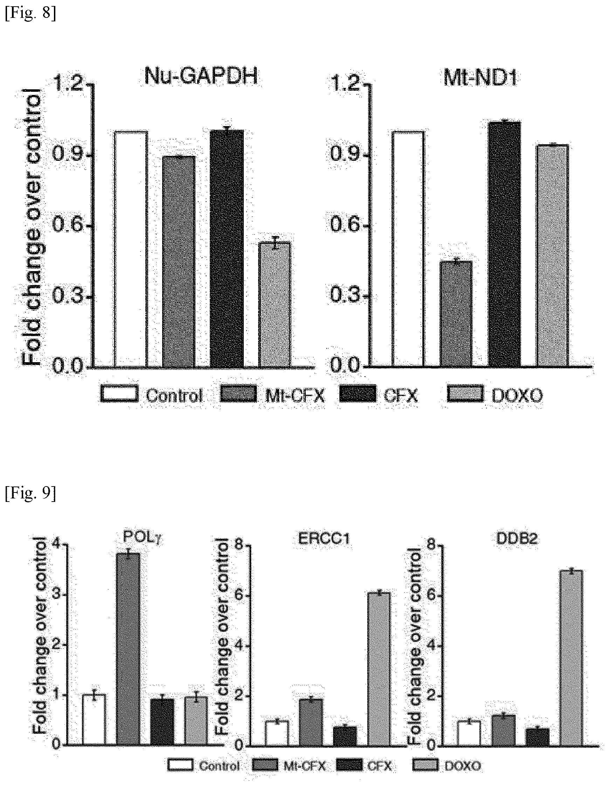

(5) Fig. 8 shows the non-toxicity of the inventive anticancer compound Ester Mt-CFX to nuclear genes by comparing the degrees of damage to nuclear DNA and mitochondrial DNA by the inventive anticancer compound Ester Mt-CFX and a commercial anticancer drug (doxorubicin (DOXO)). As a result, doxorubicin showed strong toxicity to nuclear genes whereas the inventive anticancer compound Ester Mt-CFX showed no toxicity to nuclear genes and selective toxicity to mitochondrial genes.

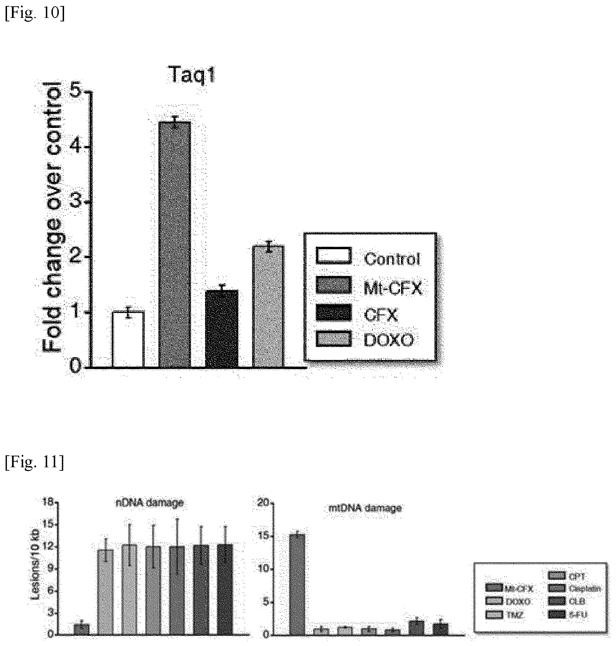

Fig. 9 shows the expression levels of DNA repair proteins after treatment with the inventive compound Ester Mt-CFX and a commercial anticancer drug (doxorubicin (DOXO)) (where POLy is a protein involved in mitochondrial gene repair and ERCC1 and DDB2 are proteins involved in nuclear gene repair). Fig. 10 reveals that unlike commercial anticancer drugs, the inventive Ester Mt-CFX targeted mitochondria, which was determined by Taq1 assay.

After treatment with the inventive Ester Mt-CFX and various anticancer drugs, DNA damage lesions were analyzed by PCR. The results are shown in Fig. 11. The commercial anticancer drugs caused many mutations in nuclear genes and Ester Mt-CFX induced mitochondrial mutations.

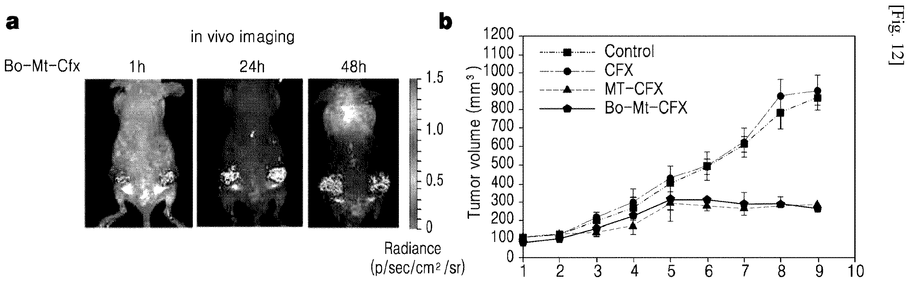

(6) The inventive fluorescent anticancer compound Ester Mt-CFX was selectively accumulated only in cancer tissues, which was confirmed by an *in vivo* experiment. Treatment with the inventive anticancer compound Ester Mt-CFX caused a significant reduction in cancer volume compared to treatment with the control, demonstrating that the inventive anticancer compound Ester Mt-CFX has a superior therapeutic effect on cancer (Fig. 12).

### Industrial Applicability

The modified antibiotic anticancer drug of the present invention selectively targets mitochondria in cancer cells to exhibit its therapeutic effect. Thus, the modified antibiotic anticancer drug can prevent cancer recurrence without causing genetic modification, unlike conventional chemotherapy for killing cancer cells by nuclear DNA damage. In addition, the modified antibiotic anticancer drug of the present invention can effectively treat malignant tumors that are difficult to treat by general anticancer therapies due to their acquired drug resistance.

## Claims

1. A modified antibiotic anticancer compound represented by Formula 1: 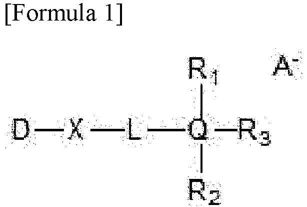 wherein D is a fluoroquinolone antibiotic; X is connected to D and is selected from O, S and NR (wherein R is selected from hydrogen, C₁-C₃₀ alkyl groups, C₆-C₃₀ aryl groups, and C₂-C₃₀ heteroaryl groups); L is selected from C₁-C₃₀ alkyl groups, C₂-C₃₀ alkenyl groups, and polyalkylene glycol groups; Q is selected from N, P, As, and Sb; R₁ to R₃ are the same as or different from each other and are each independently selected from C₁-C₃₀ alkyl groups, C₂-C₃₀ alkenyl groups, C₆-C₃₀ aryl groups, and C₂-C₃₀ heteroaryl groups; and A⁻ is an anion selected from halogen, hydroxyl, carboxylate, sulfate, sulfamate, sulfonate, phosphate, phosphonate, boronate, and (poly)ethyleneoxy anions.

2. The modified antibiotic anticancer compound according to claim 1, wherein D in Formula 1 is a fluoroquinolone antibiotic selected from flumequine, oxolinic acid, rosoxacin, cinoxacin, nalidixic acid, piromidic acid, pipemidic acid, ciprofloxacin, fleroxacin, lomefloxacin, nadifloxacin, norfloxacin, ofloxacin, pefloxacin, rufloxacin, enoxacin, balofloxacin, grepafloxacin, levofloxacin, pazufloxacin sparfloxacin, temafloxacin, tosufloxacin, clinafloxacin, gatifloxacin, moxifloxacin, sitafloxacin, prulifloxacin, besifloxacin, gemifloxacin, trovafloxacin, delafloxacin, danofloxacin, difloxacin, enrofloxacin, ibafloxacin, marbofloxacin, orbifloxacin, and sarafloxacin.

3. The modified antibiotic anticancer compound according to claim 1, wherein the compound of Formula 1 is selected from:
[Ester Mt-CFX] 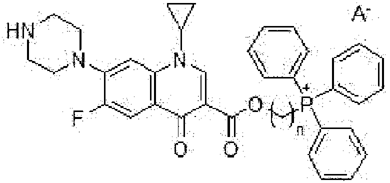
wherein A⁻ is as defined in claim 1 and n is an integer from 1 to 30; and [Amide Mt-CFX] 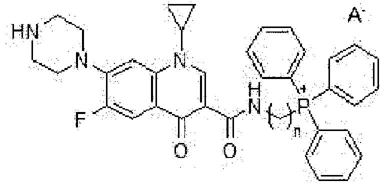
wherein A⁻ and n are as defined above.

4. The modified antibiotic anticancer compound according to claim 1, wherein the compound of Formula 1 selectively targets mitochondria in cancer cells.

5. The modified antibiotic anticancer compound according to claim 1, wherein the compound of Formula 1 inhibits the mitochondrial electron transport chain (ETC) and mitochondrial DNA synthesis in cancer cells.

6. A pharmaceutical composition for preventing and treating cancer diseases, comprising the modified antibiotic anticancer compound according to claim 1 or a salt thereof as an active ingredient.

7. The pharmaceutical composition according to claim 6, wherein the pharmaceutical composition selectively targets mitochondria in cancer cells.

8. The pharmaceutical composition according to claim 6, wherein the pharmaceutical composition inhibits the mitochondrial electron transport chain (ETC) and mitochondrial DNA synthesis in cancer cells.

9. The pharmaceutical composition according to claim 6, wherein the cancer diseases are selected from breast cancer, lung cancer, colon cancer, prostate cancer, and metastatic cancers thereof.
